(19) 

Europäisches
Patentamt
European
Patent Office
Office européen
des brevets

(11) **EP 2 410 447 B1**

(12) **EUROPEAN PATENT SPECIFICATION**

(45) Date of publication and mention
of the grant of the patent:
**21.09.2016 Bulletin 2016/38**

(21) Application number: **10753291.3**

(22) Date of filing: **16.03.2010**

(51) Int Cl.:
***G06F 19/00*** *(2011.01)*     ***G06F 19/26*** *(2011.01)*

(86) International application number:
**PCT/JP2010/001867**

(87) International publication number:
**WO 2010/106794 (23.09.2010 Gazette 2010/38)**

(54) **SYSTEM AND PROGRAM FOR ANALYZING EXPRESSION PROFILE**

SYSTEM ZUR ANALYSE VON EXPRESSIONSPROFILEN UND PROGRAMM DAFÜR

SYSTÈME D'ANALYSE D'UN PROFIL D'EXPRESSION ET PROGRAMME ASSOCIÉ

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **16.03.2009 JP 2009063273**

(43) Date of publication of application:
**25.01.2012 Bulletin 2012/04**

(73) Proprietors:
• **Meiji University
Tokyo 101-8301 (JP)**
• **The University Of Shiga Prefecture
Shiga 522-8533 (JP)**

(72) Inventors:
• **YANO, Kentaro
Kawasaki-shi
Kanagawa 214-8571 (JP)**
• **SHIMIZU, Akifumi
Hikone-shi
Shiga 522-8533 (JP)**

(74) Representative: **EP&C
P.O. Box 3241
2280 GE Rijswijk (NL)**

(56) References cited:
**JP-A- 2005 073 569**     **US-B1- 7 127 354**

• **K. YANO: "A new method for gene discovery in
large-scale microarray data", NUCLEIC ACIDS
RESEARCH, vol. 34, no. 5, 6 March 2006
(2006-03-06), pages 1532-1539, XP055103827, GB
ISSN: 0305-1048, DOI: 10.1093/nar/gkl058**
• **FELLENBERG K ET AL: "Correspondence
analysis applied to microarray data",
PROCEEDINGS OF THE NATIONAL ACADEMY
OF SCIENCES, NATIONAL ACADEMY OF
SCIENCES, US, vol. 98, no. 19, 11 September 2001
(2001-09-11), pages 10781-10786, XP002227253,
ISSN: 0027-8424, DOI: 10.1073/PNAS.181597298**
• **KENTARO YANO ET AL.: 'A new method for gene
discovery in large-scale microarray data'
NUCLEIC ACIDS RESEARCH vol. 34, no. 5, 14
March 2006, OXFORD, pages 1532 - 1539,
XP055103827**

EP 2 410 447 B1

## Description

[Technical Field]

The present invention relates to a system and program for analyzing gene expression profile data.

[Background Art]

With the advancement of genome analysis studies, a large number of novel genes having unknown functions have been identified, and it is necessary to clarify these functions. In order to obtain information regarding such functions, a gene expression pattern corresponding to the expression condition (information representing the condition under which a gene is expressed) is used.

For this reason, with EST, MPSS, SAGE, CAGE, or the like, comprehensive analyses have been performed on the expression of a large number (tens of thousands) of genes obtained from a tissue or cultured cells of a patient with a disease, or from a pathological animal model, or the like.

That is, in the case of gene analysis on the basis of the number of counts of messenger RNAs (hereinafter, referred to as mRNAs), clustering of all subject genes is carried out using gene expression profile analysis from the features of the gene expression patterns.

In general, with the use of mRNAs formed from n genes and expression frequency data of the mRNAs obtained under k independent experimental conditions, each of the n genes becomes a coordinate point having a k-dimensional feature vector in a k-dimensional feature space.

Thus, the n genes become a group of n coordinate points in the feature space by the respective feature vectors.

The expression profile analysis refers to an analysis in which the coordinate points plotted on the feature space, that is, the genes are grouped and classified into similar genes on the feature space.

With the above-described grouping, for example, an expression profile specific to a patient with a disease is obtained in which a gene expressed in a healthy person in a normal state is not expressed in a patient with a disease, the expression level increases or decreases, or the like, and thereby a distinctive gene, which is not expressed in a healthy person and is related to a disease, can be detected.

As described above, gene expression profiling is an important tool which is used to predict the function of a gene having an unknown function.

In the gene expression profile analysis, the indices of a gene expression ratio in the form of a matrix are used as data to be analyzed.

For example, gene groups to be evaluated are arranged in rows, and sample groups (target phenotypes) are arranged in columns, and the rows and columns form the gene expression profile. Specifically, the samples refer to the phenotypes which are measured by a time-course experiment with a plurality of different inspection individuals or the same individual. For example, when the expression level of 100 genes is measured with 50 individuals, the element Aij (the value of the i-th row and j-th column, $1 \leq i \leq 100$, $1 \leq j \leq 50$) of a matrix A indicates the expression level of a j-th individual with respect to an i-th gene.

The analysis of the results obtained from a large number of samples in a gene expression profile analysis requires an information processing technique to efficiently analyze the results and to find a rapidly desired gene. In the prior art, as the relevant technique, for example, special multivariable analysis, such as clustering analysis or principal component analysis, or systematic analysis is carried out (for example, see NPL 1 and NPL 2).

The gene expression profile analysis is carried out through logarithmic conversion of the gene expression level (expression ratio). Specifically, the ratio of expression levels (expression ratio, ratio) is logarithmically converted (for example, log2(ratio) or the like) into an index, and the index is mainly used for comparison of the gene expression levels between samples by a microarray experiment. For example, the reason for the logarithmic conversion is that, in the case of log2(ratio) conversion, the expression ratios of 1/4 times, 1/2 times, 1 time (equivalent expression), 2 times, and 4 times can be converted to -2, -1, 0, 1, and 2 on the same scale centering on 1 time for ease of understanding by a researcher, and the conversion is suitable for statistical analysis, or the like. However, since research institutes or researchers use 2, e, 10, and the like as the base of the logarithm, coherence is lacking, and there is a problem in that it may be impossible to directly compare data on the Web with each other.

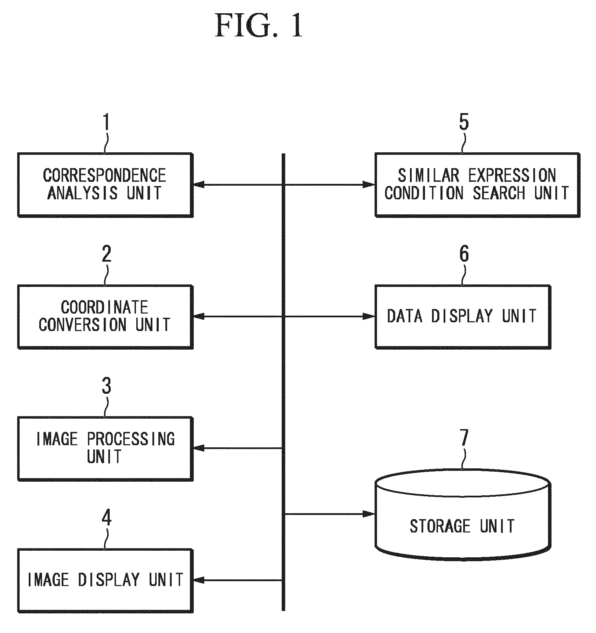

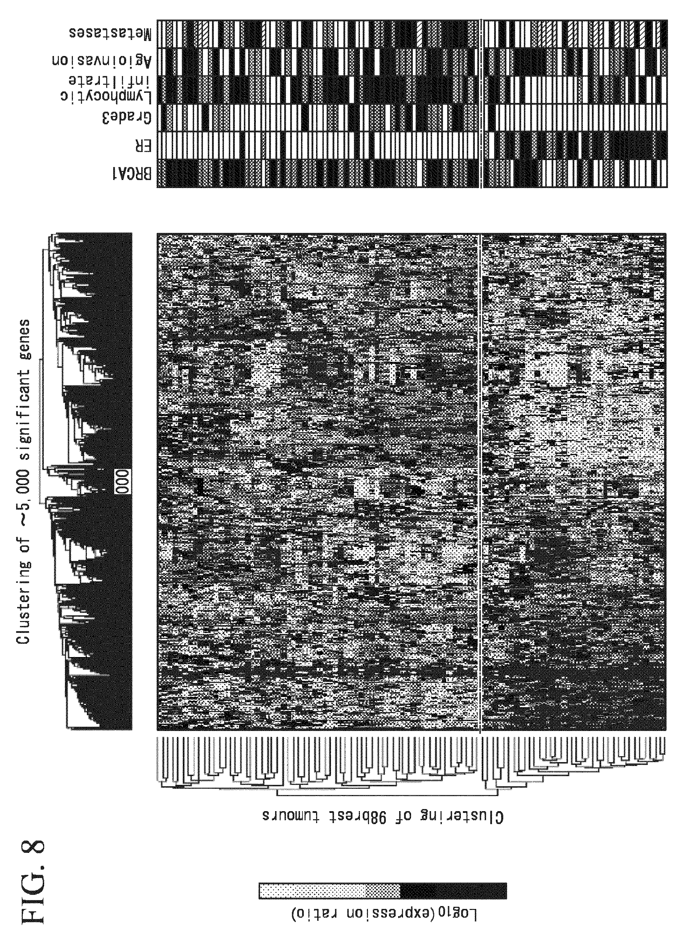

In the clustering analysis, a gene group or a sample group having a similar gene expression profile can be divided into the same cluster on the basis of a multi-dimensional feature vector. For this reason, in hierarchical clustering (for example, Ewing et al, 1999, Genome Res. 9:950-959 and the like), which is widely used in the clustering analysis, analysis by a general-purpose computer cannot be easily carried out due to an increase in the calculation amount. In general, several thousands or tens of thousands of expression genes are predicted from a large amount of current EST data. A dendrogram, which is a typical representation method of the result of the cluster analysis in gene expression patterns, is a useful method for visually recognizing the similarity between the gene expression patterns (FIG. 8 described below, and FIG. 1 in "van't Veer, L. J., Dai, H., van de Vijver, M. J., He, Y. D., Hart, A. A., Mao, M., Peterse, H. L., van der Kooy, K., Marton, M. J., Witteveen, A. T., et al. (2002) Gene expression profiling can predict the clinical outcome of breast cancer, Nature, 415, 530-536"). However, when the number of genes is equal to or greater than several thousand, it is difficult to output the entire dendrogram to a computer monitor or a printing sheet, and it takes a lot of work to analyze the result from a large-scale dendrogram.

That is, hierarchical clustering has drawbacks in that the calculation amount increases with an increase in the number of genes, in that the topology of the dendrogram is likely to change depending on a given data set, and in that the analysis time rapidly increases along with an increase in the size of a matrix, and thus a CPU and a memory of a computer are further required, and the like.

In a k-means method or an SOM (Self Organizing Maps) method, it is possible to analyze using a small number of computer resources compared with the case of the hierarchical clustering. However, at the time of analysis, it is necessary to determine the number of clusters in advance, and thus they are arbitrary methods.

In the principal component analysis method, which is an example of multi-variable analysis, it is possible to carry out calculation at high speed. However, as it is not an analysis method for a profile, expression profiles cannot be compared based on the obtained scores.

There is a problem in that it is difficult to visually recognize a large amount (ten thousand-order) of samples or gene clusters obtained by each of the above-described methods. For this reason, at present, an operation is mainly carried out to extract only a target cluster from among large-scale clusters on the basis of a Pearson's correlation coefficient or the like.

However, a researcher is unlikely to easily understand the viewer of the obtained clusters (see FIG. 8).

In the viewer shown in FIG. 8, called two-dimensional-display, genes and samples are respectively arranged vertically and horizontally (or vice versa). The color of each cell or the density of the color is visualized to represent the intensity of expression of the corresponding sample and gene.

Principal component analysis is a statistical method which directly compares the numerical values of the gene expression profile, thereby carrying out higher-speed analysis.

However, in the principal component analysis, as a result of high-speed analysis, with regard to a housekeeping gene unrelated to a phenotype to be investigated, different scores (such as coordinates) are output for each principal axis. For this reason, even when plotting is carried out on a scatter diagram, detection is not easily carried out.

[Citation List]

[Non Patent Literature]

[Non Patent Literature 1]
A Biologist's Guide to Analysis of DNA Microarray Data, YODOSHA, Steen Knudsen (author), Satoshi SHIOJIMA (translator), Gozoh TSUJIMOTO (translator), Osamu MATSUMOTO (translator)
[Non Patent Literature 2]
Sure to Get Data: Practical Manual of DNA Microarray-Basic Principle, Chip Production Technique, and Bioinformatics, YODOSHA, Yasushi Okazaki (editor), Yoshihide HAYASHIZAKIK. YANO:"A new method for gene discovery in large-scale microarray data", NUCLEIC ACIDS RESEARCH, vol. 34, no. 5, 6 March 2006 (2006-03-06), pages 1532 - 1539, discloses a gene discovery method based on correspondence analysis with an index for expression ratios [arctan (1/ratio)] and artificial marker genes.
FELLENBERG K. ET AL: "Correspondence analysis applied to microarray data", PROCEEDINGS OF THE NATIONAL ACADEMY OF SCIENCES, NATIONAL ACADEMY OF SCIENCES, US, vol. 98, no. 19, 11 September 2001 (2001-09-11), pages 10781 - 10786, discloses the applicability of correspondence analysis for the analysis of microarray data.

[Summary of Invention]

[Technical Problem]

As described above, there are various problems in the analysis methods. In particular, there is a big problem in that the analysis time (processing time) is extended, or a detection sensitivity for a minute gene expression ratio is low (a detection sensitivity for a gene involved in a quantitative trait is low).

Specifically, in the gene expression profile analysis, analysis is carried out while a large amount of data exceeding $10^3$ is processed.

However, it is difficult to rapidly calculate such a large amount of data using a general-purpose computer. As a result, the analysis time is extended.

In the prior art, in the hierarchical clustering method which is mainly used, in order to reduce or simplify the calculation time, a gene group in which the expression ratio between samples is equal to or greater than several times or equal to or smaller than several times is arbitrarily observed. This is based on the expectation that a gene whose expression level greatly changes 2 to 3 times or the like apparently affects a difference in the phenotype between samples.

On the other hand, in the hierarchical clustering method, even when the expression ratio differs significantly, a gene group with a small difference can be excluded from an analysis target.

As a result, for example, it is very difficult to detect a gene involved in a quantitative trait. That is, in this method, when a phenotype to be detected is quantitative, not qualitative, it may be impossible to detect a gene, in which the ratio of the gene expression level slightly changes, from among the genes involved in the phenotype. In other words, in the prior art method, all genes involved in a target phenotype may not be detected.

As described above, in the current analytical position, there is no viewpoint from which genes whose expression ratio slightly changes are comprehensively found. Thus, in analysis methods of the prior art (logarithmic conversion), a problem to be solved in which a detection sensitivity for a minute gene expression ratio is low do not exist.

There is a high possibility that the genes involved in the quantitative trait which are not detected in the prior art include an important novel gene. For this reason, it is essential to develop a novel large-scale analysis tool which is effective for finding genes involved in a quantitative trait.

Accordingly, the invention has been made in consideration of the problems in the prior art, and an object of the invention is to provide a system for analyzing an expression profile and a program thereof capable of rapidly analyzing a large amount of expression profile data even when using a general-purpose computer and visualizing the gene expression pattern to easily analyze to which gene of a library a novel gene is similar in function, compared to the prior art.

[Solution to Problem]

The present invention provides a system for analyzing gene expression profile data in accordance with claim 1. [0020]

In the system of the present invention, a known gene having a known function is subjected to correspondence analysis, and on the basis of the coordinate distance between the known gene and the subject gene in the n dimension, a subject gene which is similar in function to the known gene may be extracted.

In the system of the present invention, the known gene expressed by only each expression parameter is subjected to correspondence analysis as a dummy gene, and

the coordinates of the dummy gene may be set as the vertex representing an expression condition of only any of expression parameters in a figure displayed in the n dimension.

The system of the present invention may further include a similar expression condition search unit which calculates the distance between the coordinates of the dummy gene plotted in the vertex and the coordinates of the subject gene, and extracts a subject gene located in coordinates within a distance set in advance with respect to the coordinates of the vertex.

The system of the present invention may further include a data display unit which selects the subject gene and coordinates corresponding to the known gene, reads information regarding a gene plotted at a coordinate position of an image of the selected gene from the storage unit, and displays the read information.

In the system of the present invention, the coordinate conversion unit may accumulate a contribution ratio from a dimension having a high row score contribution ratio in each dimension found by the correspondence analysis unit; may compare the cumulative contribution ratio of the accumulation result with a threshold value set in advance; and may display a figure having the vertex one-dimensionally, two-dimensionally, or three-dimensionally.

The present invention provides a program for analyzing gene expression profile data in accordance with claim 5.

[Advantageous Effects of Invention]

As described above, according to the invention, by the correspondence analysis using the number of counts of mRNAs of the subject gene to be evaluated under each expression condition, each subject gene is plotted in a space (analysis space) with the coordinate value corresponding to each expression pattern and displayed in a dimension which can be displayed on the image display unit. For this reason, it is possible for the user to easily extract a gene having a shape approximate to (identical or similar to) the expression profile of an expression pattern including the number of counts of a subject gene under each expression condition, that is, a gene having a similar function from the display screen of the image display unit.

According to the invention, the expression pattern of a specific gene which is expressed under only any of expression conditions is included in a subject gene group having subject genes to be analyzed (to be evaluated), and thereby each specific gene becomes a marker representing each expression condition. Therefore, it is possible for the user to easily confirm under which expression condition each subject gene to be analyzed is strongly expressed on the display screen of the image display unit.

According to the invention, the user inputs an arbitrary distance in the space and selects a specific gene, and thereby the similar expression condition search unit extracts a subject gene included in a sphere with the distance around the specific gene as a radius. Therefore, it is possible to easily extract a subject gene having similarity based on the distance set by the user.

According to the invention, a known gene having a known function is included in a subject gene group having subject genes, and thereby each known gene becomes a marker of an expression condition representing a gene function. Therefore, it is possible for the user to easily confirm whether or not each subject gene has a function approximate to the function of the known gene on the display screen of the image display unit.

According to the invention, the display image of each gene which is displayed on the display screen of the image display unit is selected, and thereby information regarding a gene, such as the gene sequence of each gene or measurement conditions, is displayed on the display screen of the image display unit. Therefore, it is possible to easily identify unique information of the desired gene while many genes are displayed.

According to the invention, it is determined whether image display is performed one-dimensionally, two-dimensionally, or three-dimensionally on the basis of the cumulative contribution ratios of a plurality of dimensions obtained by the correspondence analysis, making it easy to view similarity on the display screen of the image display unit. (In the case of two-dimensional display, an expression condition is drawn as a line connecting vertexes as plotting positions where specific expression is made under two conditions (on two principal axes) or a polygon with the plotting positions as vertexes on a two-dimensional plane. In this case, the plotting positions become two-dimensional coordinates.)

[Brief Description of Drawings]

FIG. 1 is a block diagram showing a configuration example of a system for analyzing an expression profile according to an embodiment of the invention.

FIG. 2 is a conceptual diagram showing a configuration example of an expression data table which is stored in a storage unit 7 of FIG. 1.

FIG. 3 is a conceptual diagram showing a configuration example of a score table which is stored in the storage unit 7 of FIG. 1.

FIG. 4 is a conceptual diagram showing a configuration example of a coordinate table which is stored in the storage unit 7 of FIG. 1.

FIG. 5 is a conceptual diagram showing an image in which a pentahedron is displayed in a three-dimensional space with the display images of specific genes corresponding to five expression conditions as vertexes, the vertexes of the pentahedron are connected to each other by line segments, and character strings representing the expression conditions are displayed around the vertexes.

FIG. 6 is a conceptual diagram showing an image in which a pentahedron is displayed in a three-dimensional space with the display images of specific genes corresponding to five expression conditions as vertexes, the vertexes of the pentahedron are connected to each other by line segments, and the display images of genes are plotted.

FIG. 7 is a conceptual diagram showing an image in which a hexahedron is displayed in a three-dimensional space with the display images of specific genes corresponding to five expression conditions as vertexes, and the vertexes of the hexahedron are connected to each other by line segments, and the display images of genes are plotted.

FIG. 8 is a conceptual diagram showing a display screen of a display tool of the analysis result of a gene expression profile in an analysis system of the prior art.

[Description of Embodiments]

Hereinafter, a system for analyzing an expression profile according to an embodiment of the invention will be described with reference to the drawings. The system for analyzing an expression profile of this embodiment estimates, identifies, and predicts genes involved in a phenotype set in advance on the basis of correspondence analysis (for example, described in Noboru OHSUMI, L. Lebart, et al., "Multivariable Descriptive Analysis Method", 1994, JUSE Press Ltd.) using the number of counts under each expression condition obtained from gene expression profile data.

The term "expression profile data" refers to the expression patterns of mRNAs of a plurality of genes which are expressed in an individual sample, for example, a tissue, a cell, or the like, in other words, a data cluster including the types of genes and the respective expression levels thereof (or count values under respective expression conditions). Hereinafter, individual expression profile data is simply referred to as expression data or gene expression data. The count value under each expression condition represents the count value under each condition constituting the expression condition, and the expression pattern of the expression condition represents the pattern which is formed by the count value under each condition constituting the expression condition.

The term "phenotype" refers to an arbitrary nature involved in the characterization of each gene, and includes both a qualitative index and a quantitative index. Examples of the index involved in a disease include a disease name, cause, progress, prognosis, life expectation or pathogenesis, relapse, metastatic possibility, and the like, and are not particularly limited thereto.

An expression profile system of this embodiment is a system which can efficiently and rapidly process expression profile data regarding the number of expressions of mRNA under each expression condition from a large number of genes obtained by EST (Expressed Sequence Tag), MPSS (Massively Parallel Signature Sequencing), SAGE (Serial Analysis of Gene Expression), CAGE (Cap Analysis Gene Expression), or the like. In this embodiment, the expression conditions represent parameters for comparison with the expression level, such as gene derivation (an animal, a biological portion of the animal, or the like), environment at the time of expression, and the like.

That is, a gene involved in an arbitrary phenotype is analyzed by an expression profile experiment, in particular, correspondence analysis based on the count value under each expression condition obtained using a large number of expression data, thereby estimating the gene involved in the phenotype.

In particular, with the sequence of cDNAs obtained from cDNA clones synthesized from mRNA expressed from genes by a reverse transcription reaction using a reverse transcriptase, or the sequence of expressed genes obtained from an expressed gene fragment EST or a next-generation high-speed sequencer, it is possible to obtain information regarding growth stages, organs, tissues, or the like in which genes are expressed, as well as transcript sequence information. That is, for one or more organism species, information collection and investigation of EST sequence and derivation (growth stages or organs) are carried out, thereby enabling to search for expressed genes unique to organism species and to search for genes involved in various biological processes, such as procreation or stress response, plant photosynthesis, and absorption of nourishing water from roots. In recent years, many researchers have been conducting EST analysis of animals and plants or microorganisms, and the number of EST entries registered in the International base sequence database has exponentially increased from about 6.23 million in October 2000 to about 58.34 million in November 2008.

In recent years, large-scale expression analysis using a next-generation high-speed sequencer has been widely used. The accumulation of information obtained from EST or the next-generation high-speed sequencer enables the detailed analysis of gene expression patterns and the estimation of useful genes. Meanwhile, when extracting useful information from large-scale data, if a statistical analysis method and a tool which can be processed by even a general-purpose computer used by many researchers are not developed, it may be impossible to use accumulated basic information.

Hereinafter, a system for analyzing an expression profile of this embodiment will be described. FIG. 1 is a block diagram showing a configuration example of a system for analyzing an expression profile of this embodiment.

In FIG. 1, the system for analyzing an expression profile has a correspondence analysis unit 1, a coordinate conversion unit 2, an image processing unit 3, an image display unit 4, a similar expression condition search unit 5, a data display unit 6, and a storage unit 7. In this embodiment, the count value of the number of expressed mRNAs of each gene under each expression condition (library) is used as expression data. Thus, the count value of the mRNAs under each expression condition which is used as expression data is a numerical value obtained by any of EST, MPSS, SAGE, and CAGE.

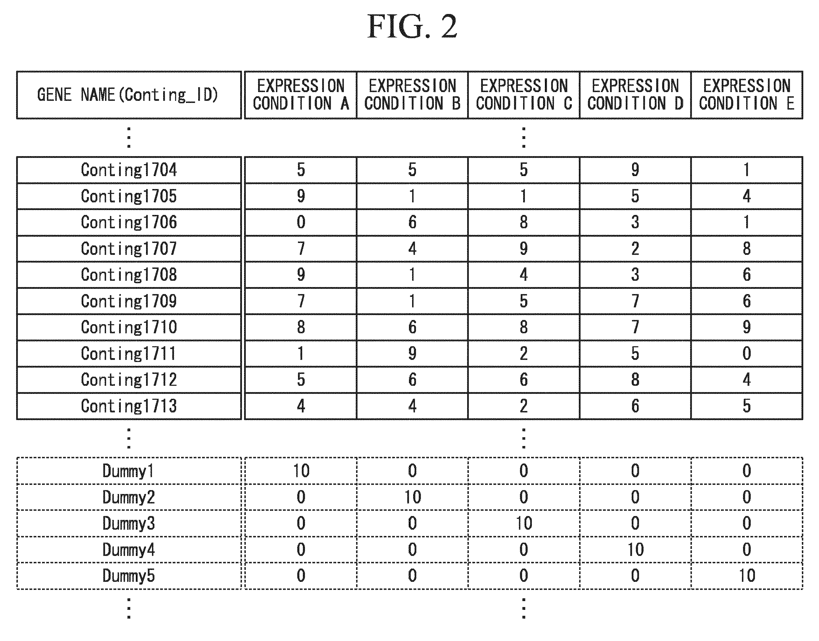

As shown in FIG. 2, the storage unit 7 stores an expression data table of the number of counts of mRNAs expressed in a gene under each of a plurality of expression conditions, for example, under each of an expression condition A, an expression condition B, an expression condition C, an expression condition D, and an expression condition E in correspondence to a gene name to be analyzed.

The correspondence analysis unit 1 sequentially reads the count value of the mRNAs under each gene expression condition as expression data from the storage unit 7, and carries out correspondence analysis on the basis of an expression pattern including the read count value as expression data under each expression condition.

The correspondence analysis in the correspondence analysis unit 1 will be simply described. The correspondence analysis is an analysis method which determines the principal axis for explaining n-dimensional data, as in the principal component analysis.

In this embodiment, the correspondence analysis unit 1 obtains one or a plurality of principal axes capable of explaining a difference in a phenotype (character or the like) using gene expression data read from the expression data table of the storage unit 7.

That is, unlike the principal component analysis simply reducing the dimension where comparison is carried out, the correspondence analysis is intended to analyze the profile of a data matrix (an expression level under an expression condition, that is, the pattern of the count value), not the amount or size of individual data, such that the expression pattern, that is, the intrinsic information amount (an expression pattern as a cluster of the count values of under each gene expression condition) of expression data as multidimensional (a plurality of expression conditions) data is not damaged.

Thus, genes having similar actions are not detected only by the expression level under any expression condition, and if the profile of the count value of the mRNAs corresponding to each expression condition is approximate, genes thereof have similar functions. For this reason, the correspondence analysis is useful for extracting a gene group having similar actions from an expression profile which is the profile of the count value under each expression condition.

As a result, genes which have expression profiles with the same expression pattern are plotted in the same coordinate space (represents the degree to which the distribution of the count value under the expression condition is identical or similar), thereby easily extracting genes or a gene group having approximate expression profiles from a large amount of expression data.

The distribution equivalence (identical or similar) in the above-described correspondence analysis can add a dummy gene (for example, a known gene for classifying function which apparently has a relevant function and also has an expression pattern as a classification criterion), which becomes an expression pattern classification index described below, to the expression data table. A case where a dummy gene is added to give meaning to a position in the distribution of the profiled gene group (or gene) will be described below.

The correspondence analysis unit 1 calculates a relative frequency in accordance with the calculation method of the correspondence analysis to obtain the expression pattern of expression data for each gene. If the element of an i-th row and a j-th column in an expression data qxp matrix of p expression conditions relating to q genes is $k_{ij}$, the correspondence analysis unit 1 divides each element $k_{ij}$ by the product of the column sum $k_i.$ of the i-th row of the following Equation (1) and the row sum

k.$_j$ of the j-th row of the following Equation (2) as conversion to the relative frequency. p and q are natural numbers equal to or greater than 2. Thus, a weight can be given to the count value under each expression condition equally for all rows and columns, and genes having similar functions can be extracted on the basis of a pattern shape which is formed by the histogram of the count value under each expression condition in the expression profile, not intensity.

[Equation 1]

$$k_i \bullet \sum_{j=1}^{p} k_{ij} \qquad \cdots (1)$$

[Equation 2]

$$k \bullet_{j} = \sum_{i=1}^{q} k_{ij} \qquad \cdots (2)$$

The correspondence analysis unit 1 obtains a transposed matrix CT from a relative frequency data matrix C having elements obtained by calculating the relative frequency, generates a matrix CT×C using the relative frequency data matrix C and the obtained transposed matrix CT, calculates the eigenvalue and eigenvector of the matrix, and obtains a plurality of principal axes for explaining a difference in expression data.

The correspondence analysis unit 1 calculates gene arrangement row scores and expression condition (library) arrangement column scores using n eigenvalues in a maximum p-gonal space (hereinafter, referred to as an analysis space, in the case of one-dimension, on a line) on the n-dimension (where n≤p) corresponding to the p expression conditions.

At this time, the dummy gene described in the expression data table of FIG. 2 is added to a gene group to be analyzed, and thereby the correspondence analysis unit 1 calculates the coordinates of the expression condition arrangement as the vertexes of the polyhedron on the basis of the dummy genes as a result of the above-described calculation processing. The coordinates of the expression condition arrangement are set row scores which are the arrangement positions of the dummy genes specific to the expression conditions. That is, the dummy genes which are specifically expressed under the respective expression conditions are plotted at the arrangement positions of each expression condition. As a result, under which expression condition (in which function) a gene to be analyzed is similar to an expression pattern is estimated based on whether or not the gene to be analyzed is similar to the expression pattern of the dummy gene set as the classification index of the expression condition.

That is, in this embodiment, a known gene (including the above-described dummy gene) having a known function is included in the correspondence analysis, thereby performing extraction processing of a subject gene which is similar in function to the known gene on the basis of the distance between the coordinate of the known gene and the coordinate of the gene to be analyzed.

That is, as a result of the correspondence analysis, in the n-dimension (corresponding to an n-dimension in the appended claims, n is a natural number), the coordinates of each gene corresponding to the coordinate values of each expression condition are obtained as scores. At this time, a more similar expression condition is scored as a coordinate having a shorter distance, and a less similar expression condition is scored as a coordinate having a longer distance. If a difference in expression data as a phenotype is explained only with one principal axis, it is on a one-dimensional line segment, and the contribution ratio of the principal axis becomes 100%.

When a change in the phenotype cannot be explained with one principal axis, for example, two principal axes (first principal axis and second principal axis) are required for explanation of a difference in the phenotype, the ratio (contribution ratio) of explanation using a two-dimensional plane on the first principal axis and the second principal axis, for example, becomes 70%, 30%, or the like.

That is, the term "contribution ratio" refers to the ratio of explanation of a change in the phenotype on a plane formed by each principal axis. The sum of the contribution ratios is referred to as a cumulative contribution ratio. At this time, the contribution ratio of the first principal axis is equal to or greater than the contribution ratio of the second principal axis. Similarly, the contribution ratio decreases in order of third and fourth principal axes. When a difference in the phenotype can be explained by the first and second principal axes, a figure representing an analysis result can be plotted one-dimensionally or two-dimensionally. When the third principal axis is required for explanation of a difference in the phenotype, a figure representing an analysis result can be plotted on a three-dimensional figure (three-dimensional space). Thus, in the correspondence analysis, the number of dimensions (that is, the number of principal axes) increases until the cumulative contribution ratio becomes 100%.

The contribution ratio is calculated from the eigenvalue which is given to each principal axis. Specifically, the ratio of the eigenvalue of each principal axis to the sum of the eigenvalues of all the principal axes becomes the contribution ratio of the relevant principal axis. For example, when principal axes (first to tenth principal axes) to a ten-dimension are obtained by the correspondence analysis for explanation of a change in the phenotype, an eigenvalue is given to each principal axis. The ratio of the eigenvalue of each principal axis to the sum of the eigenvalues of the principal axes becomes the contribution ratio, and the sum of the contribution ratios from the first principal axis to the tenth principal axis becomes the cumulative contribution ratio.

As described above, when the number of principal axes is one, a difference in the phenotype is expressed by a coordinate in a one-dimensional line segment, when the number of principal axes are two, a difference in the phenotype is expressed by a coordinate in a two-dimensional plane, when the number of principal axes is three, a difference in the phenotype is expressed by a coordinate in a three-dimensional space, ..., and when the number of principal axes is p-1, a difference in the phenotype is expressed by a coordinate in a (p-1)-dimensional space.

However, when a three-dimension (three principal axes) is calculated as a result of the correspondence analysis, the coordinate of each gene can be represented by one-dimensional, two-dimensional, or three-dimensional figure. In the case of three-dimension, the cumulative contribution ratio becomes 100% of the whole, and a difference in the phenotype can be fully explained by a three-dimensional plot diagram.

However, when the principal axes of a four-dimension or more are calculated as a result of the correspondence analysis, plotting of a four-dimension or more is actually impossible (plotting of a four-dimension or more is numerically possible, but in computer processing, normal plotting is not carried out).

For this reason, visualization with full dimension cannot be carried out. Meanwhile, for example, when the principal axes of a four-dimension or more are calculated, the cumulative contribution ratio to the third principal axis is 90%, and the contribution ratio of a subsequent principal axis is 10%, 90% of the whole can be explained even in the three-dimensional figure.

That is, determination can be made with precision of 90%. In this case, genes which are included in the remaining 10% cannot be explained in the figure. For this reason, when a four-dimensional figure is reduced to third-dimension, there is a possibility that several genes in the coordinates out of an analysis space defined by connecting each vertex in the three-dimensional space appear. That is, the principal axes obtained as n-dimension may be expressed by being reduced to m-dimension (m: natural number, and m≤n, where n≤p). For example, as described above, since image display cannot be performed in the four-dimension, the dimension is reduced to three-dimension having high-contribution-ratio three axes, and thereby display is performed.

For example, like the expression conditions of the expression data table shown in FIG. 2, when five expression conditions, i.e., the expression condition A, the expression condition B, the expression condition C, the expression condition D, and the expression condition E are used, the dummy genes which are specifically expressed under each expression condition are included, and thereby the phenotype of each gene is explained using the principal axes of a five-dimension or less. In this example, the correspondence analysis unit 1 obtains row scores, which become four pieces of coordinate data of score 1, score 2, score 3, and score 4 corresponding to four-dimension, as the coordinates in which each gene is plotted.

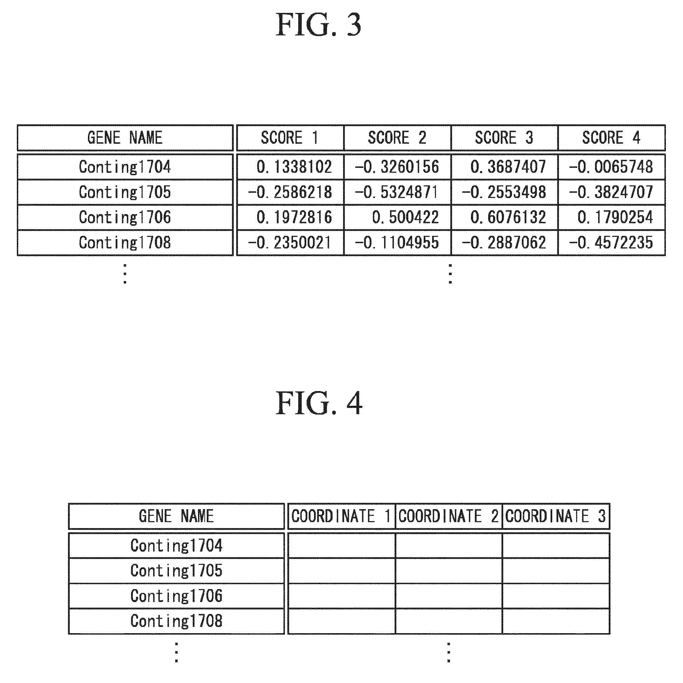

The correspondence analysis unit 1 writes the scores for each principal axis (principal axis 1, principal axis 2, principal axis 3, and principal axis 4) into a score table shown in FIG. 3 in correspondence to each gene in the storage unit 7.

Since the four-dimension or more cannot be displayed, the correspondence analysis unit 1 sets the display dimension to maximum three-dimension, and outputs the score 1, the score 2, and the score 3 to the coordinate conversion unit 2 in a descending order of the contribution ratio of the principal axis along with the contribution ratios of each dimension.

If the score 1, the score 2, and the score 3 to the three-dimension as a result of the correspondence analysis are input along with the contribution ratios, the coordinate conversion unit 2 compares the contribution ratio of one-dimension, the cumulative contribution ratio obtained by adding the contribution ratios of one-dimension and two-dimension, and the cumulative contribution ratio obtained by adding the contribution ratios of one-dimension, two-dimension, and three-dimension with a set contribution ratio set in advance, and sets a combination of dimensions exceeding the set contribution ratio as the dimension of a display space. The contribution ratio of one-dimension is highest, and the contribution ratio decreases in order of two-dimension and three-dimension.

For example, when the cumulative contribution ratio of only one-dimension, that is, the contribution ratio exceeds the set contribution ratio, the coordinate conversion unit 2 arranges genes on a line, which connects two vertexes drawn on a two-dimensional plan, with a one-dimensional score. When a gene is closer to any one vertex on the line, this indicates that the gene is expressed strongly under the relevant expression condition. In this case, when the number of types of the expression conditions exceeds two (is three or more), the coordinates of a plurality of expression conditions overlap each other.

When the cumulative contribution ratio obtained by adding the contribution ratios of one-dimension and two-dimension exceeds the set contribution ratio, the coordinate conversion unit 2 arranges genes in a two-dimensional space with one-dimensional and two-dimensional scores. In this case, a polygonal analysis plane having the vertexes as the arrangement coordinates of each expression condition (specific genes) is formed in a two-dimensional plane. On the two-dimensional plane within the polygon, when a gene is closer to any one vertex of the polygon, this indicates that the gene is expressed strongly under the relevant expression condition. The one-dimensional score is used as the coordinate value of the x coordinate, and the two-dimensional score is used as the coordinate value of the y coordinate.

When the cumulative contribution ratio obtained by adding the contribution ratios of one-dimension and two-dimension does not exceed the set contribution ratio set in advance, the coordinate conversion unit 2 arranges genes in a three-dimensional space with one-dimensional, two-dimensional, and three-dimensional scores. In this case, in the three-dimensional space, a polyhedral analysis space having vertexes as the arrangement coordinates of each expression condition (specific genes) is formed. In the three-dimensional space within the polyhedron, when a gene is closer to any one vertex of the polyhedron, this indicates that the gene is expressed strongly under the relevant expression condition. The one-dimensional score is used as the coordinate value of the x coordinate, the two-dimensional score is used as the coordinate value of the y coordinate, and the three-dimensional score is used as the coordinate value of the z coordinate.

For example, when the display image of each gene is displayed in the three-dimensional space, if data of each dimension shown in FIG. 3, that is, the score 1, the score 2, and the score 3 are input, the coordinate conversion unit 2 calculates the absolute values of a positive score and a negative score for each gene on each score, detects the maximum value, divides the score of each gene by the maximum value, and sets the result as a coordinate value. Thus, the display image and the vertex of each gene are plotted in a real-number range in the coordinate space of the x, y, and z axes.

In this way, the score of each gene is normalized by divided by the maximum value of the scores of each dimension, processing is performed for equalizing the weight of each dimension, and when there are n vertexes of the expression conditions, in this case, four vertexes to be displayed in the display space of the image display unit 4, the coordinate values of relative coordinates of the coordinates of the four vertexes with the coordinate of any one vertex as the origin and the coordinates of each gene in an analysis space surrounded by the vertexes are converted to the coordinate values of absolute coordinates in the display space of the image display unit 4.

The coordinate conversion unit 2 writes the values of the coordinates (for example, coordinate 1: x axis, coordinate 2: y axis, and coordinate 3: z axis) for arranging the genes in correspondence to each gene in the storage unit 7 with the configuration of a coordinate table shown in FIG. 4. The coordinate conversion unit 2 writes the value of the coordinates, in which each expression condition is plotted as a vertex, into the storage unit 7 in correspondence to each expression condition.

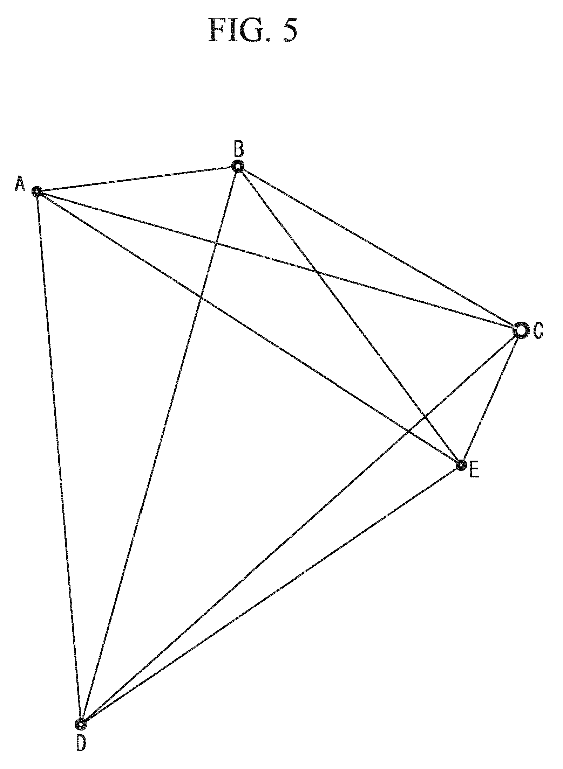

As shown in FIG. 5, the image processing unit 3 reads the coordinate values of the vertexes corresponding to each expression condition from the storage unit 7 and displays five vertexes of the expression condition A, the expression condition B, the expression condition C, the expression condition D, and the expression condition E and a line segment connecting each vertex in the display space of the image display unit 4. At this time, the image processing unit 3 displays a character string indicating an expression condition, to which each vertex corresponds, around each vertex. For example, the image processing unit 3 displays a character string "A" indicating an expression condition around the vertex corresponding to the expression condition A. The character string is stored in the storage unit 7 in correspondence to each expression condition, when the image processing unit 3 draws a figure with the expression conditions of FIG. 5 as vertexes, is read from the storage unit 7 in correspondence to each expression condition, and is displayed around the vertex of the corresponding expression condition.

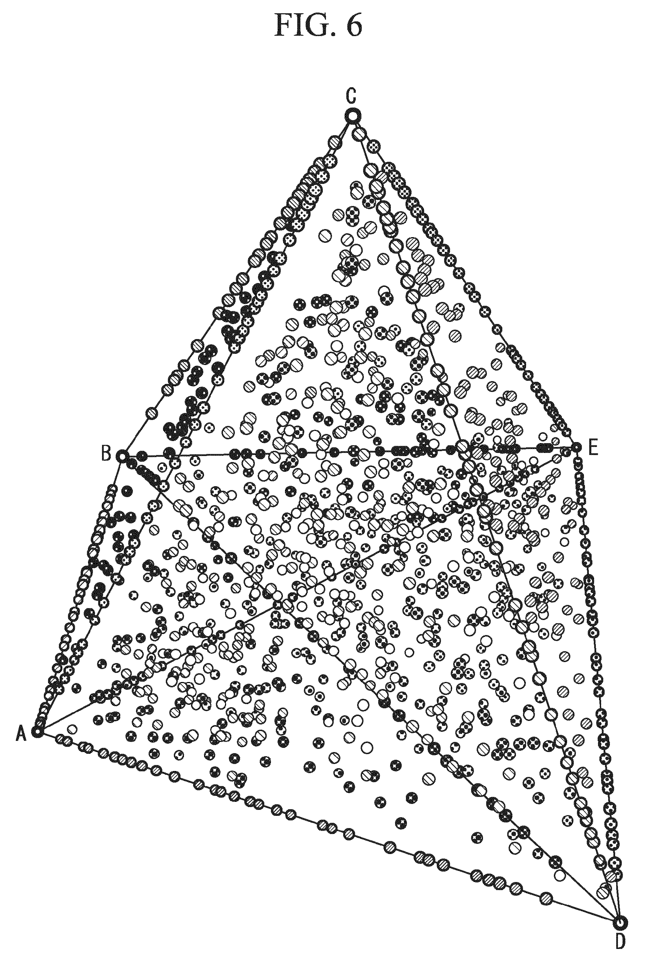

The image processing unit 3 sequentially reads the coordinate values of each gene from the coordinate table of FIG. 4, and as shown in FIG. 6, displays the display images indicating each gene (for example, spherical dots, cubic dots, characters, or the like) in an analysis space of a polyhedron with each expression condition as vertexes, that is, in this example, a polyhedron having maximum five vertexes based on the coordinate values corresponding to the genes. FIGS. 5 and 6 show an example where the principal axes of a three-dimension are used, and a pentahedron is used as a polyhedron in a three-dimensional space.

In the display processing, as described above, genes which are expressed specifically under the corresponding expression conditions or dummy genes are plotted at each vertex corresponding to each expression condition.

The expression data table which is stored in the storage unit 7 stores dummy gene identification information indicating a relevant gene being a dummy gene for each dummy gene in correspondence to the gene name (when forming an expression table, the user sets dummy gene identification information for each dummy gene).

When arranging the dummy genes, the image processing unit 3 displays the display images of the dummy genes in colors different from other genes being displayed.

On a line segment which connects the vertexes, genes which are expressed in correspondence to the expression conditions of the two vertexes connected by the line segment are plotted.

For example, on a line segment which connects the vertexes corresponding to the expression condition A and the expression condition C, genes which are expressed under the expression condition A and the expression condition C are plotted. The positions where the genes are plotted on each line segment are set such that the expression pattern of each gene plotted on the line segment is plotted in the coordinate of a position approximate to the vertex, at which one dummy gene having a more similar expression pattern is plotted, with respect to the dummy genes plotted at the vertexes corresponding to two expression conditions.

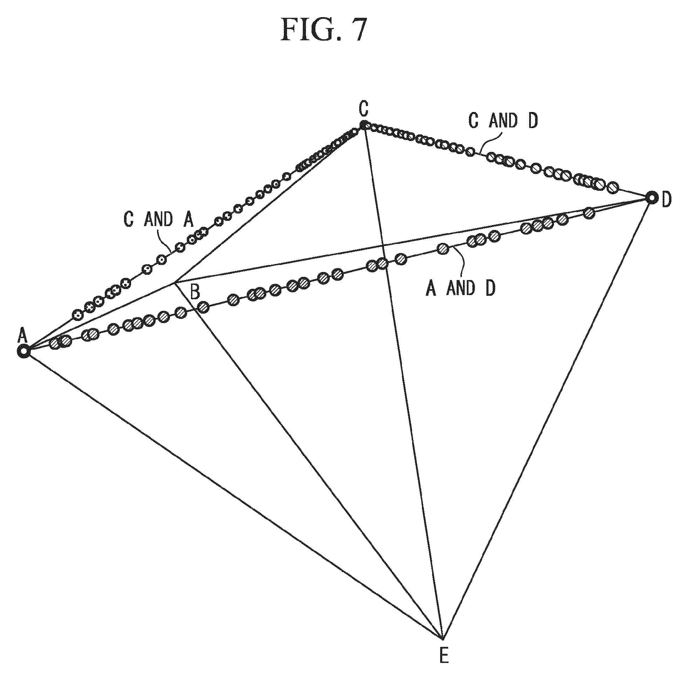

In FIG. 7, genes are plotted only on a line which connects the expression condition A and the expression condition C, a line which connects the expression condition A and the expression condition D, and a line which connects the expression condition C and the expression condition D.

That is, in the analyzed gene group, there are only genes which are expressed only under the expression condition A and the expression condition C, genes which are expressed only under the expression condition A and the expression condition D, and genes which are expressed only under the expression condition C and the expression condition D.

The genes which are expressed under three expression conditions of the expression condition A, the expression condition C, and the expression condition D are plotted on a surface (plane) formed by the vertexes corresponding to the expression condition A, the expression condition C, and the expression condition D in the polyhedron formed by the five vertexes, and are plotted at the positions approximate to the vertexes having a more similar expression pattern on the plane.

The genes which are expressed under four expression conditions of the expression condition A, the expression condition B, the expression condition C, and the expression condition D are plotted in a space (three-dimensional space) of a polyhedron formed by the vertexes corresponding to the expression condition A, the expression condition B, the expression condition C, and the expression condition D in the polyhedron formed by the five vertexes, and are plotted at the position approximate to the vertexes having a more similar expression pattern in the three-dimensional space.

The image processing unit 3 displays the display images of the genes plotted on a line segment connecting each vertex in different colors between each line segment.

Similarly, the image processing unit 3 displays the display images of the genes plotted on a surface connecting each vertex in colors different from the display images plotted on the line segments and other surfaces of the polyhedron.

The image processing unit 3 displays the display images plotted in the internal space of the polyhedron connecting the vertexes in colors different from the display images plotted on the line segments and the surfaces of the polyhedron and in the internal space of another polyhedron.

The image processing unit 3 performs processing for rotating an image being displayed in an arbitrary direction, for example, at a set angle with the x axis, the y axis, or the z axis as a rotation axis, reversing an image horizontally, or reversing an image vertically in accordance with the user's settings.

The data display unit 6 reads the gene name of a gene plotted in a coordinate corresponding to coordinate data of a gene selected by user's clicking or the like on the display screen of the image display unit 4 from the coordinate table stored in the storage unit 7 in correspondence to the coordinate value.

The data display unit 6 reads information regarding the gene corresponding to the gene name from the expression data table and displays the information around the coordinate of the gene having the gene name.

When a plurality of genes are plotted in the same coordinate, and a gene in the coordinate is selected by the user, the data display unit 6 reads a plurality of gene names plotted in the coordinate from the coordinate table on the basis of the coordinate and displays the gene names around the selected coordinate in the form of a list.

If there is a gene with information for reference from among the gene names in the list, and the gene is selected by the user, the data display unit 6 reads information regarding the gene corresponding to the gene name of the gene selected in the list from the expression data table of the storage unit 7 and displays the read information regarding the gene around the selected coordinate.

From the value of a distance input by the user using a mouse, a keyboard, or the like (not shown) and the coordinate of a selected gene (for example, a dummy gene), the similar expression condition search unit 5 changes the color of a gene in a sphere with the input distance as radius to the color of another gene plotted.

As a result, it is possible for the user to easily extract a gene which is similar to a gene selected by the user, that is, a gene (or a gene group) having a target expression pattern.

An $\chi$ square distance can be used as a statistically significant position from the dummy gene, the line segment, or the surface of the polyhedron. With the use of the $\chi$ square distance, it is possible to calculate a significant distance with a significance level of 1% or the like.

For example, a gene or a gene group which is expressed specifically under one expression condition can be defined as a gene (gene group) located within the $\chi$ square distance from each vertex.

When an expression pattern of interest is complicated, that is, the histogram of the count value under each expression condition has a complicated distribution, a known gene having a corresponding expression pattern is appended (added) to the expression data table, thereby easily searching the coordinate of the known gene having an expression pattern of interest and a gene having a similar function at a shorter distance from the known gene than other genes.

It can be estimated that a gene which is similar in expression pattern to a known gene having a known biological function has the same function or is involved in gene expression control.

Thus, a known gene in which a function by an expression pattern (by the count value under each expression condition) is detected in advance is added to the expression data table stored in the storage unit 7, and thereby the correspondence analysis unit 1 calculates the coordinate of the known gene and the row scores of the known gene on the basis of the expression pattern of the count value under each expression condition in the same manner as other genes.

The similar expression condition search unit 5 extracts genes within a spherical space with a distance set in advance, for example, the above-described $\chi$ square distance based on the coordinate of the known gene as a radius, that is, genes having a similar expression pattern (similar function) to the known gene.

Thus, the user can easily detect the genes having a function approximate to the known gene. As described above, although a dummy gene which uses a coordinate as a vertex is included in the known gene according to large classification, the dummy gene follows small classification in that the dummy gene is expressed only under one expression condition in a certain expression pattern.

In the correspondence analysis, each plot of the row scores and the column scores obtained as the analysis result can be plotted (biplot) on the same line in the case of one-dimension, on the same plane in the case of two-dimension, or on the same space in the case of three-dimension.

That is, as described above, the coordinate conversion unit 2 converts the row scores representing the gene arrangement and the column scores representing the expression condition arrangement to the coordinates in the coordinate table of FIG. 4.

The image processing unit 3 displays the genes and the display images of the expression conditions sequentially read from the coordinate table on the display screen of the image display unit 4.

In an image displayed on the display screen of the image display unit 4, if a distance between an expression condition approximate to a coordinate obtained by correspondence analysis and a gene to be analyzed is small, this means that there is a high relationship.

For example, there is a high possibility that a gene (group) which is around an expression condition representing the phenotype (expression profile) such as a patient with caner is involved in the phenotype. The similar expression condition search unit 5 extracts a gene (group), which is estimated to have high relationship, using the $\chi$ square distance (described above) from the coordinate of the expression condition.

A program for realizing the functions of the system for analyzing an expression profile in FIG. 1 may be recorded into a computer-readable recording medium, and the program recorded in the recording medium may be read and executed on a computer system, thereby performing expression profile analysis processing. The "computer system" includes an OS and hardware, such as a peripheral device. The "computer system" also includes a WWW system which includes a homepage provision environment (or display environment). The "computer-readable recording medium" refers to a portable medium, such as a flexible disc, a magneto-optical disc, a ROM, or a CD-ROM, or a storage device, such as a hard disk incorporated in a computer system. The "computer-readable recording medium" also includes a device for holding a program for a given time, such as an internal volatile memory (RAM) of a computer system serving as a server or a client when the program is transmitted through a network, such as Internet, or a communication link, such as a telephone line.

The program may be transmitted from a computer system which stores the program in a storage device or the like to another computer system through a transmission medium or by transmission waves in the transmission medium. The "transmission medium" which transmits a program refers to a medium having a function of transmitting information, and includes a network (communication network), such as Internet, or a communication link (communication line), such as a telephone line. The program may realize some of the above-described functions. The program may realize the above-described functions in combination with a program already recorded in a computer system, that is, the program may be a differential file (differential program).

[Industrial Applicability]

The invention provides a system for analyzing an expression profile in which a large number of expression profile data obtained by a next-generation high-speed sequencer, a similar experimental technique, or the like is analyzed at high speed by a general-purpose computer, gene expression patterns are visualized, thereby easily analyzing to which gene a novel gene is similar in function. Therefore, the invention is very useful for industrial applications.

[Reference Signs List]

1: correspondence analysis unit
2: coordinate conversion unit
3: image processing unit
4: image display unit
5: similar expression condition search unit
6: data display unit
7: storage unit

**Claims**

1. A system for analyzing gene expression profile data, the system comprising:

a storage unit which stores the number of counts of mRNAs having been expressed from a subject gene to be evaluated as expression data in correspondence to the subject gene under each of a plurality of gene expression conditions;
a correspondence analysis unit which reads the expression data from the storage unit for each subject gene and carries out correspondence analysis on the basis of the number of counts under each expression condition in expression data;
a coordinate conversion unit which converts n-dimensional scores obtained by the correspondence analysis to coordinate values for m-dimensionally arranging each subject gene, wherein n and m are natural numbers, and m≤n; and
an image processing unit which carries out plotting along the corresponding coordinate values for each gene to display the result on an image display unit,
wherein a known gene having a known function is subjected to the correspondence analysis, and on the basis of the coordinate distance between the known gene and the subject gene in the n dimension, a subject gene which is similar in function to the known gene is extracted, and
wherein the known gene expressed by only each expression parameter is subjected to the correspondence analysis as a dummy gene, and the coordinates of the dummy gene are set as the vertex representing an expression condition of only any of expression parameter in a figure displayed on the n dimension.

2. The system according to Claim 1, further comprising:

a similar expression condition search unit which calculates the distance between the coordinates of the dummy gene plotted in the vertex and the coordinates of the subject gene, and extracts a subject gene located in coordinates within a distance set in advance with respect to the coordinates of the vertex.

3. The system according to Claim 1 or 2, further comprising:

a data display unit which selects the subject gene and coordinates corresponding to the known gene, reads information regarding a gene plotted at a coordinate position of an image of the selected gene from the storage unit, and displays the read information.

4. The system according to any one of Claims 1 to 3, wherein the coordinate conversion unit accumulates a contribution ratio from a dimension having a high row score contribution ratio in each dimension found by the correspondence analysis unit, compares the cumulative contribution ratio of the accumulation result with a threshold value set in advance, and displays a figure having the vertex one-dimensionally, two-dimensionally, or three-dimensionally.

5. A program for analyzing gene expression profile data, the program allowing a computer to execute:

correspondence analysis in which a correspondence analysis unit reads, from a storage unit which stores the number of counts of mRNAs expressed from a subject gene to be evaluated as expression data under each of a plurality of gene expression conditions in correspondence to the subject gene, expression data of each subject gene, and carries out correspondence analysis on the basis of the number of counts under each expression condition in expression data;
coordinate conversion in which a coordinate conversion unit converts n-dimensional scores obtained by the correspondence analysis to coordinate values for m-dimensionally arranging each subject gene, wherein n and m are natural numbers, and m≤n; and
image processing in which an image processing unit carries out plotting along the corresponding coordinate values for each gene to display the result on an image display unit,
wherein a known gene having a known function is subjected to the correspondence analysis, and on the basis of the coordinate distance between the known gene and the subject gene in the n dimension, a subject gene which is similar in function to the known gene is extracted, and
wherein the known gene expressed by only each expression parameter is subjected to the correspondence analysis as a dummy gene, and the coordinates of the dummy gene are set as the vertex representing an expression condition of only any of expression parameter in a figure displayed on the n dimension.

**Patentansprüche**

1. System zum Analysieren von Genexpressionsprofildaten, wobei das System umfasst:

eine Speichereinheit, welche die Anzahl von Zählungen von mRNAs speichert, die von einem auszuwertenden Subjekt-Gen in Korrespondenz zu dem Subjekt-Gen unter jeder einer Mehrzahl von Genexpressionsbedingungen als Expressionsdaten exprimiert wurden;
eine Korrespondenzanalyseneinheit, welche die Expressionsdaten aus der Speichereinheit für jedes Subjekt-Gen ausliest und in den Expressionsdaten eine Korrespondenzanalyse auf der Basis der Anzahl von Zählungen unter jeder Expressionsbedingung ausführt;
eine Koordinatenumwandlungseinheit, welche n-dimensionale Treffer, die durch die Korrespondenzanalyse erhalten werden, in Koordinatenwerte umwandelt, um jedes Subjekt-Gen m-dimensional anzuordnen, wobei n und m natürliche Zahlen sind, und m ≤ n ist; und

eine Bildbearbeitungseinheit, welche ein Plotten entlang der korrespondierenden Koordinatenwerte für jedes Gen ausführt, um das Ergebnis auf einer Bildanzeigeeinheit wiederzugeben, wobei ein bekanntes Gen mit einer bekannten Funktion der Korrespondenzanalyse unterzogen wird und auf der Basis des Koordinatenabstands zwischen dem bekannten Gen und dem Subjekt-Gen in der n-Dimension ein Subjekt-Gen extrahiert wird, welches in seiner Funktion gleich dem bekannten Gen ist, und

wobei das durch nur jeden Expressionsparameter exprimierte bekannte Gen als Dummy-Gen der Korrespondenzanalyse unterzogen wird und die Koordinaten des Dummy-Gens als der Scheitelpunkt gesetzt werden, der eine Expressionsbedingung von nur einem beliebigen Expressionsparameter in einer in der n-Dimension wiedergegebenen Figur repräsentiert.

**2.** System nach Anspruch 1, ferner mit:

einer Ähnliche-Expressionsbedingung-Sucheinheit, welche den Abstand zwischen den Koordinaten des in dem Scheitelpunkt geplotteten Dumnry-Gens und den Koordinaten des Subjekt-Gens berechnet und ein mit seinen Koordinaten innerhalb eines voreingestellten Abstands in Bezug zu den Koordinaten des Scheitelpunkts liegendes Subjekt-Gen extrahiert.

**3.** System nach Anspruch 1 oder 2, ferner mit:

einer Datenanzeigeeinheit, welche das Subjekt-Gen und Koordinaten, die mit dem bekannten Gen korrespondieren, auswählt, Informationen betreffend eines Gens liest, die an einer Koordinatenposition eines Bildes des aus der Speichereinheit ausgewählten Gens geplottet sind, und die gelesene Information wiedergibt.

**4.** System nach einem der Ansprüche 1 bis 3, in welchem die Koordinatenumwandlungseinheit ein Beitragsverhältnis aus einer Dimension sammelt, die ein durch die Korrespondenzanalyseeinheit in jeder Dimension gefundenes hohes Zeilentreffer-Beitragsverhältnis hat, das kumulative Beitragsverhältnis des Sammelergebnisses mit einem im Voraus eingestellten Schwellenwert vergleicht und eine Figur mit dem Scheitelpunkt eindimensional, zweidimensional oder dreidimensional wiedergibt.

**5.** Programm zum Analysieren von Genexpressionsprofildaten, wobei das Programm einem Computer ermöglicht, auszuführen:

eine Korrespondenzanalyse, in welcher eine Korrespondenzanalyseeinheit aus einer Speichereinheit, welche die Anzahl von Zählungen von mRNAs speichert, die von einem auszuwertenden Subjekt-Gen als Expressionsdaten unter jeder einer Mehrzahl von Genexpressionsbedingungen in Korrespondenz mit dem Subjekt-Gen exprimiert wurden, Expressionsdaten jedes Subjekt-Gens ausliest, und in den Expressionsdaten eine Korrespondenzanalyse auf der Basis der Anzahl von Zählungen unter jeder Expressionsbedingung ausführt;

eine Koordinatenumwandlungsanalyse, in welcher eine Koordinatenumwandlungseinheit n-dimensionale Treffer, die durch die Korrespondenzanalyse erhalten werden, in Koordinatenwerte umwandelt, um jedes Subjekt-Gen m-dimensional anzuordnen, wobei n und m natürliche Zahlen sind, und m ≤ n ist; und

eine Bildbearbeitung, in welcher eine Bildbearbeitungseinheit ein Plotten entlang der korrespondierenden Koordinatenwerte für jedes Gen ausführt, um das Ergebnis auf einer Bildanzeigeeinheit wiederzugeben, wobei ein bekanntes Gen mit einer bekannten Funktion der Korrespondenzanalyse unterzogen wird und auf der Basis des Koordinatenabstands zwischen dem bekannten Gen und dem Subjekt-Gen in der n-Dimension ein Subjekt-Gen extrahiert wird, welches in seiner Funktion gleich dem bekannten Gen ist, und

wobei das durch nur jeden Expressionsparameter exprimierte bekannte Gen der Korrespondenzanalyse als ein Dummy-Gen unterzogen wird und die Koordinaten des Dummy-Gens als der Scheitelpunkt gesetzt werden, der eine Expressionsbedingung von nur einem beliebigen Expressionsparameter in einer in der n-Dimension wiedergegebenen Figur repräsentiert.

## Revendications

**1.** Système pour analyser les données du profil d'expression d'un gène, le système comprenant :

une unité de stockage qui stocke le nombre d'occurrences d'ARNm qui ont été exprimées à partir d'un gène en question devant être évalué en tant que données d'expression en correspondance avec le gène en question dans chacune d'une pluralité de conditions d'expression du gène ;

une unité d'analyse de correspondance qui lit les données d'expression de l'unité de stockage pour chaque gène en question et réalise une analyse de correspondance en se basant sur le nombre d'occurrences dans chaque condition d'expression des données d'expression ;

une unité de conversion de coordonnées qui convertit les scores en n dimensions obtenus par l'analyse de correspondance en valeurs de coordonnées afin de représenter chaque gène en m dimensions, où n et m sont des nombres naturels, et m ≤ n ; et

une unité de traitement d'image qui réalise un tracé le long des valeurs de coordonnées correspondantes pour chaque gène afin d'afficher le résultat sur une unité d'affichage d'image,

où un gène connu ayant une fonction connue est soumis à l'analyse de correspondance et, en se basant sur la distance des coordonnées entre le gène connu et le gène en question dans la dimension n, un gène en question qui possède une fonction similaire au gène connu est extrait, et

où le gène connu exprimé par seulement chaque paramètre d'expression est soumis à l'analyse de correspondance en tant que gène factice, et les coordonnées du gène factice sont définies comme le vertex représentant une condition d'expression de seulement l'un quelconque des paramètres d'expression sur une figure affichée sur la dimension n.

2. Système selon la revendication 1, comprenant en outre :

une unité de recherche de condition d'expression similaire qui calcule la distance entre les coordonnées du gène factice tracé dans le vertex et les coordonnées du gène en question, et extrait un gène en question dont les coordonnées sont localisées à une certaine distance définie à l'avance par rapport aux coordonnées du vertex.

3. Système selon la revendication 1 ou 2, comprenant en outre :

une unité d'affichage de données qui sélectionne le gène en question et les coordonnées correspondant au gène connu, lit les informations relatives à un gène tracé à une position de coordonnées d'une image du gène sélectionné à partir de l'unité de stockage, et affiche les informations lues.

4. Système selon l'une quelconque des revendications 1 à 3,

dans lequel l'unité de conversion de coordonnées accumule un rapport de contribution à partir d'une dimension présentant un rapport de contribution ayant un score de ligne élevé dans chaque dimension identifiée par l'unité d'analyse de correspondance, compare le rapport de contribution cumulatif du résultat d'accumulation avec une valeur seuil définie à l'avance, et affiche une figure comportant le vertex en une dimension, deux dimensions, ou trois dimensions.

5. Programme pour analyser les données du profil d'expression d'un gène, le programme permettant à un ordinateur d'exécuter :

une analyse de correspondance dans laquelle une unité d'analyse de correspondance lit, à partir d'une unité de stockage qui stocke le nombre d'occurrences d'ARNm exprimées à partir d'un gène en question devant être évalué en tant que données d'expression dans chacune d'une pluralité de conditions d'expression du gène en correspondance avec le gène en question, les données d'expression de chaque gène en question, et réalise une analyse de correspondance en se basant sur le nombre d'occurrences dans chaque condition d'expression des données d'expression ;

une conversion des coordonnées dans laquelle une unité de conversion de coordonnées convertit les scores en n dimensions obtenus par l'analyse de correspondance en valeurs de coordonnées afin de représenter chaque gène en question en m dimensions, où n et m sont des nombres naturels, et m ≤ n ; et

un traitement d'image dans lequel une unité de traitement d'image réalise un tracé le long des valeurs de coordonnées correspondantes pour chaque gène afin d'afficher le résultat sur une unité d'affichage d'image,

où un gène connu ayant une fonction connue est soumis à l'analyse de correspondance et, en se basant sur la distance de coordonnées entre le gène connu et le gène en question dans la dimension n, un gène en question qui possède une fonction similaire au gène connu est extrait, et

où le gène connu exprimé par seulement chaque paramètre d'expression est soumis à l'analyse de correspondance en tant que gène factice, et les coordonnées du gène factice sont définies comme le vertex représentant une condition d'expression de seulement l'un quelconque des paramètres d'expression sur une figure affichée sur la dimension n.

# FIG. 1

# FIG. 2

| GENE NAME(Conting_ID) | EXPRESSION CONDITION A | EXPRESSION CONDITION B | EXPRESSION CONDITION C | EXPRESSION CONDITION D | EXPRESSION CONDITION E |
|---|---|---|---|---|---|
| ⋮ | ⋮ | | ⋮ | | |
| Conting1704 | 5 | 5 | 5 | 9 | 1 |
| Conting1705 | 9 | 1 | 1 | 5 | 4 |
| Conting1706 | 0 | 6 | 8 | 3 | 1 |
| Conting1707 | 7 | 4 | 9 | 2 | 8 |
| Conting1708 | 9 | 1 | 4 | 3 | 6 |
| Conting1709 | 7 | 1 | 5 | 7 | 6 |
| Conting1710 | 8 | 6 | 8 | 7 | 9 |
| Conting1711 | 1 | 9 | 2 | 5 | 0 |
| Conting1712 | 5 | 6 | 6 | 8 | 4 |
| Conting1713 | 4 | 4 | 2 | 6 | 5 |
| ⋮ | | | ⋮ | | |
| Dummy1 | 10 | 0 | 0 | 0 | 0 |
| Dummy2 | 0 | 10 | 0 | 0 | 0 |
| Dummy3 | 0 | 0 | 10 | 0 | 0 |
| Dummy4 | 0 | 0 | 0 | 10 | 0 |
| Dummy5 | 0 | 0 | 0 | 0 | 10 |
| ⋮ | | | ⋮ | | |

EP 2 410 447 B1

# FIG. 3

| GENE NAME | SCORE 1 | SCORE 2 | SCORE 3 | SCORE 4 |
|-----------|---------|---------|---------|---------|
| Conting1704 | 0.1338102 | -0.3260156 | 0.3687407 | -0.0065748 |
| Conting1705 | -0.2586218 | -0.5324871 | -0.2553498 | -0.3824707 |
| Conting1706 | 0.1972816 | 0.500422 | 0.6076132 | 0.1790254 |
| Conting1708 | -0.2350021 | -0.1104955 | -0.2887062 | -0.4572235 |
| ⋮ | | ⋮ | | |

# FIG. 4

| GENE NAME | COORDINATE 1 | COORDINATE 2 | COORDINATE 3 |
|-----------|--------------|--------------|--------------|
| Conting1704 | | | |
| Conting1705 | | | |
| Conting1706 | | | |
| Conting1708 | | | |
| ⋮ | | ⋮ | |

# FIG. 5

# FIG. 6

# FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **EWING et al.** *Genome Res.,* 1999, vol. 9, 950-959
- **VAN'T VEER, L. J. ; DAI, H. ; VAN DE VIJVER, M. J. ; HE, Y. D. ; HART, A. A. ; MAO, M. ; PETERSE, H. L. ; VAN DER KOOY, K. ; MARTON, M. J. ; WITTEVEEN, A. T. et al.** Gene expression profiling can predict the clinical outcome of breast cancer. *Nature,* 2002, vol. 415, 530-536
- **STEEN KNUDSEN.** A Biologist's Guide to Analysis of DNA Microarray Data. YODOSHA
- Sure to Get Data: Practical Manual of DNA Microarray-Basic Principle, Chip Production Technique, and Bioinformatics. YODOSHA
- **YOSHIHIDE HAYASHIZAKI K. YANO.** A new method for gene discovery in large-scale microarray data. *NUCLEIC ACIDS RESEARCH,* 06 March 2006, vol. 34, 1532-1539
- **FELLENBERG K. et al.** Correspondence analysis applied to microarray data. *PROCEEDINGS OF THE NATIONAL ACADEMY OF SCIENCES, NATIONAL ACADEMY OF SCIENCES,* 11 September 2001, vol. 98 (19), 10781-10786
- **NOBORU OHSUMI, L. LEBART et al.** Multivariable Descriptive Analysis Method. JUSE Press Ltd, 1994